# EUROPEAN PATENT APPLICATION

(11) **EP 4 847 529 A1**
(43) Date of publication of application: **02.12.2026**
(21) Application number: 25744707.8
(22) Date of filing: 24.01.2025
(51) Int. Cl.: C07K 16/28, C12N 15/13, A61K 39/395, A61P 35/00

(54) **ANTI-B7H3 ANTIBODY AND USE THEREOF**

(30) Priority: 25.01.2024 CN 202410110647
(71) Applicant: Shandong Simcere Zaiming Biopharmaceutical Co., Ltd., Yantai, Shandong 264006 (CN)
(72) Inventor: CHENG, Guangcun, Shanghai 201318 (CN); FU, Yayuan, Shanghai 201318 (CN); TANG, Renhong, Shanghai 201318 (CN); LI, Jiangmei, Beijing 100176 (CN); GUO, Yanan, Beijing 102609 (CN)
(74) Representative: Müller-Boré & Partner Patentanwälte PartG mbB
(86) International application number: PCT/CN2025/074524
(87) International publication number: WO 2025/157244

(57) **Abstract**

The present application discloses an antibody specifically binding to B7H3 or an antigen-binding fragment thereof, a nucleic acid encoding same, a recombinant vector, a host cell, a preparation method, a pharmaceutical composition, and a use in the treatment of tumor diseases, which can be used for the development of B7H3-targeted therapeutic drugs and the development of detection reagents.

## Description

The present disclosure claims priority to the Chinese Patent Application No. 202410110647.3 entitled "ANTI-B7H3 ANTIBODY AND USE THEREOF", filed with the China National Intellectual Property Administration on January 25, 2024, which is incorporated herein by reference in its entirety.

### TECHNICAL FIELD

The present disclosure relates to the field of antibodies, and in particular, to an anti-B7H3 antibody.

### BACKGROUND

B7-H3 is a type I transmembrane protein, also known as CD276, and is a member of the B7 ligand family. Early studies suggest that B7H3 is a T cell co-stimulatory factor that plays a regulatory role during T cell activation and IFNγ production. Some other studies suggest that B7H3 plays an inhibitory role in adaptive immunity, possibly through different receptors. However, the receptors of B7H3 are not clear at present. The human B7H3 protein has 534 amino acids in full length and exists in two subtypes, B7H3-4Ig and B7H3-2Ig. The B7H3-4Ig subtype comprises 2 pairs of immunoglobulin variable region IgV and immunoglobulin constant region IgC-like domains, and is the major subtype in humans. The B7H3-2Ig subtype comprises only one pair of IgV and IgC domains. B7H3 is widely expressed in normal tissues but at limited expression levels, remaining at relatively low levels, e.g., in the liver, colon, and prostate. However, the expression of B7H3 is relatively high in tumor tissues and is closely related to the progression, survival, and prognosis of tumor patients. It has been found that B7H3 is highly expressed in various tumor tissues such as small cell lung cancer, prostate cancer, renal cancer, colorectal cancer, and lung squamous cell carcinoma. In tumor tissues, B7H3 is expressed in both tumor cells and interstitial cells, such as tumor vascular endothelial cells, pericytes, and fibroblasts. This suggests that B7H3 plays an important role in cancer progression, migration, invasion, anti-apoptosis, metabolism, and angiogenesis. Although the physiological function of B7H3 has not yet been fully elucidated, it is still expected to become an attractive target for tumor treatment due to its relatively high expression levels and the relatively high number of positive patients. Currently, therapeutic modalities targeting B7H3 are already under development, such as ADCC-enhanced monoclonal antibodies, CD3 bispecific antibodies, CART, and antibody-drug conjugates (ADCs). However, no B7H3-targeted drugs have been approved for marketing so far.

### SUMMARY

The present disclosure provides a novel antibody or an antigen-binding fragment thereof specifically binding to B7H3, thereby providing a novel tumor therapy.

In a first aspect of the present disclosure, provided is an antibody or an antigen-binding fragment thereof specifically binding to B7H3, comprising a heavy chain variable region (VH) and a light chain variable region (VL), wherein
(1) the heavy chain variable region comprises an HCDR1, an HCDR2, and an HCDR3, wherein the HCDR1, the HCDR2, and the HCDR3 are an HCDR1, an HCDR2, and an HCDR3 of a VH domain set forth in SEQ ID NO. 12; and
(2) the light chain variable region comprises an LCDR1, an LCDR2, and an LCDR3, wherein the LCDR1, the LCDR2, and the LCDR3 are an LCDR1, an LCDR2, and an LCDR3 of a VL domain set forth in SEQ ID NO. 13.

In some specific embodiments, the HCDR1, the HCDR2, and the HCDR3 have the amino acid sequences set forth in SEQ ID NOs. 14-16, SEQ ID NOs. 20-22, or SEQ ID NOs. 26-28 or a sequence combination having 1, 2, 3, or more amino acid insertions, deletions, and/or substitutions compared with the amino acid sequences set forth in SEQ ID NOs. 14-16, SEQ ID NOs. 20-22, or SEQ ID NOs. 26-28, as determined according to the Kabat, Chothia, or IMGT numbering scheme.

In some specific embodiments, the LCDR1, the LCDR2, and the LCDR3 have the amino acid sequences set forth in SEQ ID NOs. 17-19, SEQ ID NOs. 23-25, or SEQ ID NOs. 29-31 or a sequence combination having 1, 2, 3, or more amino acid insertions, deletions, and/or substitutions compared with the amino acid sequences set forth in SEQ ID NOs. 17-19, SEQ ID NOs. 23-25, or SEQ ID NOs. 29-31, as determined according to the Kabat, Chothia, or IMGT numbering scheme.

In some specific embodiments, the antibody or the antigen-binding fragment thereof comprises the following LCDR1, LCDR2, LCDR3, HCDR1, HCDR2, and HCDR3:
(1) according to the Kabat numbering scheme, LCDR1-3 and HCDR1-3 having the amino acid sequences set forth in SEQ ID NOs. 14-19 or a sequence combination having 1, 2, 3, or more amino acid insertions, deletions, and/or substitutions compared with the amino acid sequences set forth in SEQ ID NOs. 14-19;
(2) according to the IMGT numbering scheme, LCDR1-3 and HCDR1-3 having the amino acid sequences set forth in SEQ ID NOs. 20-25 or a sequence combination having 1, 2, 3, or more amino acid insertions, deletions, and/or substitutions compared with the amino acid sequences set forth in SEQ ID NOs. 20-25;
(3) according to the Chothia numbering scheme, LCDR1-3 and HCDR1-3 having the amino acid sequences set forth in SEQ ID NOs. 26-31 or a sequence combination having 1, 2, 3, or more amino acid insertions, deletions, and/or substitutions compared with the amino acid sequences set forth in SEQ ID NOs. 26-31.

In some specific embodiments, the sequence of the heavy chain variable region comprises the sequence set forth in SEQ ID NO. 12 or a sequence having 80%, 85%, 90%, 95%, 96%, 97%, 98%, 99%, or higher identity thereto; the sequence of the light chain variable region comprises the sequence set forth in SEQ ID NO. 13 or a sequence having 80%, 85%, 90%, 95%, 96%, 97%, 98%, 99%, or higher identity thereto.

In some specific embodiments, the antibody or the antigen-binding fragment thereof is fully human-derived.

In some specific embodiments, the antibody or the antigen-binding fragment thereof is capable of specifically binding to a human or monkey B7H3 protein.

In some specific embodiments, the antibody or the antigen-binding fragment thereof may further comprise any constant region sequence of a human or murine antibody IgG 1, IgG2, IgG3, IgG4, IgA, IgM, IgE, or IgD, preferably a constant region sequence of a human or murine antibody IgG 1, IgG2, IgG3, or IgG4, or a constant region sequence of a human or murine antibody IgG1, IgG2, IgG3, or IgG4 having a mutation; further, the antibody or the antigen-binding fragment thereof is further conjugated to a therapeutic agent or a tracer; preferably, the therapeutic agent is selected from a radioisotope, a chemotherapeutic agent, a cytotoxic agent, or an immunomodulator, and the tracer is selected from a radiocontrast medium, a paramagnetic ion, a metal, a fluorescent label, a chemiluminescent label, an ultrasound contrast agent, and a photosensitizer; more preferably, the cytotoxic agent is selected from alkaloids, methotrexate, anthracyclines (doxorubicin), pyrrolobenzodiazepine (PBD), gemcitabine, cytarabine, tegafur, ifosfamide, dacarbazine, and oxaliplatin; more preferably, the cytotoxic agent is a taxane.

In some specific embodiments, the antigen-binding fragment is selected from one or more of a F(ab')₂, a Fab', a Fab, an Fv, an scFv, a nanobody, or an affibody.

In a second aspect of the present disclosure, provided is a multispecific antigen-binding molecule comprising the antibody or the antigen-binding fragment thereof according to the first aspect, wherein preferably, the multispecific antigen-binding molecule further comprises an antibody or an antigen-binding fragment thereof specifically binding to an antigen other than B7H3 or binding to a B7H3 epitope different from a B7H3 epitope to which the antibody or the antigen-binding fragment thereof according to the first aspect binds;
preferably, the antigen other than B7H3 is selected from the group consisting of: (1) a tumor-specific antigen (TSA) or a tumor-associated antigen (TAA); (2) an immune checkpoint; and (3) a target for recruiting and/or activating an immune cell;
preferably, the multispecific antigen-binding molecule may be bispecific, trispecific, or tetraspecific;
preferably, the multispecific antigen-binding molecule may be divalent, trivalent, tetravalent, pentavalent, or hexavalent.

In a third aspect of the present disclosure, provided is a chimeric antigen receptor (CAR) comprising at least an extracellular antigen-binding domain, a transmembrane domain, and an intracellular signaling domain, wherein the extracellular antigen-binding domain comprises the antibody or the antigen-binding fragment thereof according to the first aspect.

In a fourth aspect of the present disclosure, provided is an immune effector cell expressing the chimeric antigen receptor according to the third aspect, or comprising a nucleic acid fragment encoding the chimeric antigen receptor, wherein preferably, the immune effector cell is selected from a T cell, a natural killer cell (NK cell), a natural killer T cell (NKT cell), a double negative T cell (DNT cell), a monocyte, a macrophage, a dendritic cell, or a mast cell, and the T cell is selected from a cytotoxic T cell, a regulatory T cell, or a helper T cell; preferably, the immune effector cell is an auto-immune effector cell or an allogeneic immune effector cell.

In a fifth aspect of the present disclosure, provided is an isolated nucleic acid fragment encoding the antibody or the antigen-binding fragment thereof according to the first aspect, the multispecific antigen-binding molecule according to the second aspect, or the chimeric antigen receptor according to the third aspect.

In a sixth aspect of the present disclosure, provided is a vector comprising the nucleic acid fragment. In a seventh aspect of the present disclosure, provided is a host cell comprising the vector, wherein preferably, the cell is a prokaryotic cell or a eukaryotic cell, such as a bacterium (*E. coli*), a fungus (yeast), an insect cell, or a mammalian cell (a CHO cell line or a 293T cell line).

In an eighth aspect of the present disclosure, provided is a method for preparing the antibody or the antigen-binding fragment thereof according to the first aspect or the multispecific antigen-binding molecule, comprising culturing the host cell and isolating an antibody or an antigen-binding fragment thereof expressed by the host cell, or isolating a multispecific antigen-binding molecule expressed by the host cell.

In a ninth aspect of the present disclosure, provided is a method for preparing the immune effector cell, comprising introducing a nucleic acid fragment encoding the CAR according to the third aspect into the immune effector cell, wherein optionally, the method further comprises initiating expression of the CAR by the immune effector cell.

In a tenth aspect of the present disclosure, provided is a pharmaceutical composition comprising the antibody or the antigen-binding fragment thereof according to the first aspect, or the multispecific antigen-binding molecule according to the second aspect, or the immune effector cell according to the fourth aspect, or the nucleic acid fragment according to the fifth aspect, or the vector according to the sixth aspect, or the host cell according to the seventh aspect, or an antibody or an antigen-binding fragment thereof or a multispecific antigen-binding molecule prepared by the method according to the eighth aspect, or an immune effector cell prepared by the method according to the ninth aspect, wherein optionally, the pharmaceutical composition further comprises a pharmaceutically acceptable carrier, diluent, or adjuvant.

In an eleventh aspect of the present disclosure, provided is use of the antibody or the antigen-binding fragment thereof according to the first aspect, or the multispecific antigen-binding molecule according to the second aspect, or the immune effector cell according to the fourth aspect, or the nucleic acid fragment according to the fifth aspect, or the vector according to the sixth aspect, or the host cell according to the seventh aspect, or an antibody or an antigen-binding fragment thereof or a multispecific antigen-binding molecule prepared by the method according to the eighth aspect, or an immune effector cell prepared by the method according to the ninth aspect, or the pharmaceutical composition according to the tenth aspect in preparing a medicament for preventing and/or treating a tumor, wherein the tumor is selected from a solid tumor, a hematologic tumor, or a B7H3-expressing infiltrative cancer;
preferably, the tumor is selected from melanoma, prostate cancer, renal cell carcinoma, lung cancer (e.g., non-small cell lung cancer (NSCLC)), and other solid tumors.

In a twelfth aspect of the present disclosure, provided is a method for preventing and/or treating a tumor, comprising administering to a patient in need thereof an effective amount of the antibody or the antigen-binding fragment thereof according to the first aspect, or the multispecific antigen-binding molecule according to the second aspect, or the immune effector cell according to the fourth aspect, or the nucleic acid fragment according to the fifth aspect, or the vector according to the sixth aspect, or the host cell according to the seventh aspect, or an antibody or an antigen-binding fragment thereof or a multispecific antigen-binding molecule prepared by the method according to the eighth aspect, or an immune effector cell prepared by the method according to the ninth aspect, or the pharmaceutical composition according to the tenth aspect, wherein the tumor is selected from a solid tumor, a hematologic tumor, or a B7H3-expressing infiltrative cancer;
preferably, the tumor is selected from melanoma, prostate cancer, renal cell carcinoma, lung cancer (e.g., non-small cell lung cancer (NSCLC)), and other solid tumors.

In a thirteenth aspect of the present disclosure, provided is a kit comprising the antibody or the antigen-binding fragment thereof according to the first aspect, or the multispecific antigen-binding molecule according to the second aspect, or the immune effector cell according to the fourth aspect, or the nucleic acid fragment according to the fifth aspect, or the vector according to the sixth aspect, or the host cell according to the seventh aspect, or an antibody or an antigen-binding fragment thereof or a multispecific antigen-binding molecule prepared by the method according to the eighth aspect, or an immune effector cell prepared by the method according to the ninth aspect, or the pharmaceutical composition according to the tenth aspect.

In a fourteenth aspect of the present disclosure, provided is a method for detecting expression of B7H3 *in vitro,* comprising contacting a test sample with the antibody or the antigen-binding fragment thereof according to the first aspect under conditions that allow the antibody or the antigen-binding fragment thereof and B7H3 to form a complex, wherein preferably, the method further comprises detecting formation of the complex and indicating the presence or expression level of B7H3 in the sample.

In a fifteenth aspect of the present disclosure, provided is use of the antibody or the antigen-binding fragment thereof according to the first aspect in preparing a B7H3 detection reagent.

### Terms and Definitions

Unless otherwise defined in the present disclosure, scientific and technical terms used in correlation with the present disclosure shall have the meanings that are commonly understood by those of ordinary skill in the art.

Furthermore, unless otherwise stated herein, terms used in the singular form herein shall include the plural form, and vice versa. More specifically, as used in this specification and the appended claims, unless otherwise clearly indicated, the singular forms "a", "an", and "the" include referents in the plural form.

The terms "include", "comprise", and "have" herein are used interchangeably and are intended to indicate the inclusion of a solution, implying that there may be elements other than those listed in the solution. Meanwhile, it will be appreciated that the descriptions "include", "comprise", and "have" used herein also provide the solution of "consist of".

The term "and/or" used herein includes the meanings of "and", "or", and "all or any other combination of elements linked by the term".

The term "B7H3" herein is a member of the B7-CD28 superfamily and is expressed on an antigen-presenting cell. B7-H3 binds to T cells, but a B7-H3 counter-receptor on the surface of such T cells has not been fully characterized. The main human form of B7H3 includes two extracellular tandem IgV-IgC domains (i.e., IgV-IgC-IgV-IgC). Although it was initially thought to include only 2 Ig domains (IgV-IgC), a four-immunoglobulin extracellular domain variant ("4Ig-B7-H3") has been identified and has been found to be a more common human form of the protein. The native murine form (2Ig) and the human 4Ig form exhibit similar functions. The 4Ig-B7-H3 molecule inhibits NK cell-mediated lysis of cancer cells. B7H3 mRNA expression has been found in the heart, kidney, testis, lung, liver, pancreas, prostate, colon, and osteoblasts.

The term "specifically bind to" herein means that an antigen-binding molecule (e.g., an antibody) specifically binds to an antigen and substantially identical antigens, generally with high affinity, but does not bind to unrelated antigens with high affinity. The affinity is generally reflected in an equilibrium dissociation constant (KD), where a relatively low KD indicates a relatively high affinity. In the case of antibodies, high affinity generally means having a KD of about 10⁻⁶ M or less, 10⁻⁷ M or less, about 10⁻⁸ M or less, or about 10⁻⁹ M or less. The KD is calculated as follows: KD = Kd/Ka, where Kd represents the dissociation rate, and Ka represents the association rate. The equilibrium dissociation constant KD can be measured by methods well known in the art, such as surface plasmon resonance (e.g., Biacore) or equilibrium dialysis.

The term "antigen-binding molecule" herein is used in its broadest sense and refers to a molecule that specifically binds to an antigen. Illustratively, the antigen-binding molecule includes, but is not limited to, an antibody or an antibody mimetic. The "antibody mimetic" refers to an organic compound or a binding domain that is capable of specifically binding to an antigen, but is not structurally related to an antibody. Illustratively, the antibody mimetic includes, but is not limited to, an affibody, an affitin, an affilin, a designed ankyrin repeat protein (DARPin), a nucleic acid aptamer, or a Kunitz domain peptide.

The term "antibody" herein is used in its broadest sense and refers to a polypeptide or a combination of polypeptides that comprises a sufficient sequence from an immunoglobulin heavy chain variable region and/or a sufficient sequence from an immunoglobulin light chain variable region to be capable of specifically binding to an antigen. The "antibody" herein encompasses various forms and various structures as long as they exhibit the desired antigen-binding activity. The "antibody" herein includes alternative protein scaffolds or artificial scaffolds having grafted complementarity determining regions (CDRs) or CDR derivatives. Such scaffolds include antibody-derived scaffolds comprising mutations introduced to, for example, stabilize the three-dimensional structure of the antibody, and fully synthetic scaffolds comprising, for example, biocompatible polymers. See, e.g., Korndorfer, I. P., Beste, G. & Skerra, A. (2003). Proteins, 53, 121-129.; Roque, A. C. A., Lowe, C. R. & Taipa, M. A. Antibodies and genetically engineered related molecules: production and purification. Biotechnol. Prog., 20, 639-654 (2004), the contents of which are incorporated herein in their entirety. Such scaffolds may also include non-antibody-derived scaffolds, such as scaffold proteins known in the art to be useful for grafting CDRs, including but not limited to tenascin, fibronectin, peptide aptamers, and the like.

The term "antibody" includes an intact antibody and any antigen-binding fragment (i.e., "antigen-binding moiety") or single chain thereof. The "antibody" includes a glycoprotein or an antigen-binding moiety thereof comprising at least two heavy (H) chains and two light (L) chains that are linked together by disulfide bonds. Each heavy chain consists of a heavy chain variable region (abbreviated herein as VH) and a heavy chain constant region. The heavy chain constant region consists of three domains: CH1, CH2, and CH3. Each light chain consists of a light chain variable region (abbreviated herein as VL) and a light chain constant region. The light chain constant region consists of one domain: CL. The VH region and the VL region can be further subdivided into hypervariable regions called complementarity determining regions (CDRs), which are scattered among regions that are more conserved, termed framework regions (FRs). Each VH or VL consists of three CDRs and four FRs, which are arranged from the amino-terminus to the carboxyl-terminus in the following order: FR1, CDR1, FR2, CDR2, FR3, CDR3, and FR4. The variable regions of the heavy and light chains comprise binding domains that can interact with antigens. The constant regions of the antibody may mediate the binding of the immunoglobulin to a host tissue or factor, and the host tissue or factor includes various cells (e.g., effector cells) of the immune system and the first component (C1q) of the classical complement system. The heavy chain constant regions of immunoglobulins differ in their amino acid composition and arrangement, and thus in their antigenicity. Accordingly, the "immunoglobulin" herein can be divided into five classes, or isotypes of immunoglobulins, i.e., IgM, IgD, IgG, IgA, and IgE, with their corresponding heavy chains being µ, δ, γ, α, and ε chains, respectively. The Ig of the same class may also be divided into different subclasses according to the differences in the amino acid composition of the hinge regions and the number and location of disulfide bonds in the heavy chains. For example, IgG may be divided into IgG1, IgG2, IgG3, and IgG4, and IgA may be divided into IgA1 and IgA2. Light chains are divided into κ chains or λ chains according to differences in the constant regions. Each of the five classes of Ig may have a κ chain or λ chain.

The "antibody" herein also includes antibodies that do not comprise a light chain, e.g., heavy-chain antibodies (HCAbs) produced by *Camelidae* species, e.g., *Camelus dromedarius, Camelus bactrianus, Lama glama, Lama guanicoe,* and *Vicugna pacos,* as well as immunoglobulin (Ig) new antigen receptors (IgNARs) found in *Chondrichthyes,* e.g., sharks.

The term "antibody" herein may be derived from any animal, including but not limited to human and non-human animals, and the non-human animals may be selected from primates, mammals, rodents, and vertebrates, such as *Camelidae* species, *Lama glama, Lama guanicoe, Vicugna pacos,* sheep, rabbits, mice, rats, or *Chondrichthyes* (e.g., sharks).

The term "heavy chain antibody" herein refers to an antibody lacking a light chain of a conventional antibody. The term specifically includes, but is not limited to, homodimeric antibodies comprising a VH antigen-binding domain and CH2 and CH3 constant domains in the absence of a CH1 domain.

As used herein, the term "nanobody" refers to a heavy chain antibody naturally lacking a light chain present in a camel, and the cloning of its variable region can give a single domain antibody only consisting of a heavy chain variable region (also called VHH (variable domain of heavy chain of heavy chain antibody)), which is the smallest functional antigen-binding fragment.

The terms "nanobody" and "single domain antibody (sdAb)" herein have the same meaning and are used interchangeably, and refer to a single domain antibody consisting of only one heavy chain variable region constructed by cloning a variable region of a heavy chain antibody, which is the smallest antigen-binding fragment having the complete function. Generally, a single domain antibody consisting of only one heavy chain variable region is constructed by acquiring a heavy chain antibody naturally lacking a light chain and a heavy chain constant region 1 (CH1) and then cloning the variable region of the antibody heavy chain.

For further description of "heavy chain antibody" and "nanobody", see: Hamers-Casterman et al., Nature, 1993; 363; 446-8; a review article (Reviews in Molecular Biotechnology, 74: 277-302, 2001) by Muyldermans; and the following patent applications mentioned as general background art: WO 94/04678, WO 95/04079, and WO 96/34103; WO94/25591, WO 99/37681, WO 00/40968, WO 00/43507, WO 00/65057, WO 01/40310, WO 01/44301, EP 1134231, and WO 02/48193; WO97/49805, WO 01/21817, WO 03/035694, WO 03/054016, and WO 03/055527; WO 03/050531; WO 01/90190; WO03/025020; and WO 04/041867, WO 04/041862, WO 04/041865, WO 04/041863, WO 04/062551, WO 05/044858, WO 06/40153, WO 06/079372, WO 06/122786, WO 06/122787, and WO 06/122825, as well as other prior art mentioned in these applications. (The above contents are incorporated herein in their entirety).

The term "multispecific" herein refers to the ability of an antibody or an antigen-binding fragment thereof to bind to, e.g., different antigens or to at least two different epitopes on the same antigen. Thus, the terms such as "bispecific", "trispecific", and "tetraspecific" refer to the number of different epitopes to which an antibody can bind. For example, conventional monospecific IgG antibodies have two identical antigen-binding sites (paratopes) and therefore can only bind to the same epitope (rather than to different epitopes). In contrast, multispecific antibodies have at least two different types of paratopes/binding sites and can therefore bind to at least two different epitopes. As used herein, the "complementarity determining region" refers to the antigen-binding site of an antibody. In addition, a single "specificity" may refer to one, two, three, or more identical complementarity determining regions in a single antibody (the actual number of complementarity determining regions/binding sites in a single antibody molecule is referred to as the "valency"). For example, a single native IgG antibody is monospecific and divalent, since it has two identical paratopes. Accordingly, a multispecific antibody comprises at least two (different) complementarity determining regions/binding sites. Thus, the term "multispecific antibody" refers to an antibody having more than one paratope and the ability to bind to two or more different epitopes. The term "multispecific antibody" particularly includes bispecific antibodies as defined above, but typically also includes proteins, e.g., antibodies and scaffolds specifically binding to three or more different epitopes, i.e., antibodies having three or more paratopes/binding sites.

The term "valent" herein refers to the presence of a specified number of binding sites in an antibody/antigen-binding molecule. Thus, the terms "monovalent", "divalent", "tetravalent", and "hexavalent" refer to the presence of one binding site, two binding sites, four binding sites, and six binding sites, respectively, in an antibody/antigen-binding molecule.

"Full-length antibody", "complete antibody", and "intact antibody" herein are used interchangeably and refer to an antibody having a structure substantially similar to that of a natural antibody.

"Antigen-binding fragment" and "antibody fragment" herein are used interchangeably and do not have the entire structure of an intact antibody, but comprise only a portion of the intact antibody or a variant of the portion that has the ability to bind to an antigen. Illustratively, the "antigen-binding fragment" or "antibody fragment" herein includes, but is not limited to, a Fab, a F(ab')₂, a Fab', a Fab'-SH, an Fd, an Fv, an scFv, a diabody, and a single domain antibody.

The term "chimeric antibody" herein refers to an antibody having a variable region sequence of an immunoglobulin derived from one organism (e.g., rat, mouse, rabbit, or *Vicugna pacos*) and a constant region of an immunoglobulin derived from a different organism (e.g., human). Methods for producing chimeric antibodies are known in the art. See, e.g., Patent US5807715A; Morrison, 1985, Science, 229(4719): 1202-1207. Transfectomas Provide Novel Chimeric Antibodies; Gillies et al., J Immunol Methods, 1989 Dec 20, 125(1-2): 191-202, the contents of which are incorporated herein in their entirety.

The term "humanized antibody" herein refers to a genetically engineered non-human-derived antibody that has an amino acid sequence modified to increase the homology to the sequence of a human-derived antibody. Generally, all or part of the CDRs of a humanized antibody are derived from a non-human-derived antibody (donor antibody), and all or part of the non-CDRs (e.g., variable region FRs and/or constant regions) are derived from a human immunoglobulin (receptor antibody). The humanized antibody generally retains or partially retains the desired properties of the donor antibody, including but not limited to, antigen specificity, affinity, reactivity, the ability to increase the activity of immune cells, the ability to enhance an immune response, or the like.

The term "fully human-derived antibody" herein refers to an antibody having variable regions in which both the FRs and CDRs are derived from human germline immunoglobulin sequences. Furthermore, if the antibody comprises constant regions, the constant regions are also derived from human germline immunoglobulin sequences. The fully human-derived antibody herein may include amino acid residues that are not encoded by human germline immunoglobulin sequences (e.g., mutations introduced by random or site-specific mutagenesis *in vitro* or by somatic mutations *in vivo*). However, the "fully human-derived antibody" herein does not include antibodies in which CDR sequences derived from the germline of another mammalian species (e.g., mouse) have been grafted onto human framework sequences.

The term "variable region" herein refers to a region of a heavy or light chain of an antibody involved in the binding of the antibody to an antigen. The "heavy chain variable region" is used interchangeably with "VH" and "HCVR", and the "light chain variable region" is used interchangeably with "VL" and "LCVR". The heavy and light chain variable domains of natural antibodies generally have similar structures, each domain containing four conserved framework regions (FRs) and three hypervariable regions (HVRs). See, e.g., Kindt et al., Kuby Immunology, 6th ed., W. H. Freeman and Co., p. 91 (2007), the content of which is incorporated herein in its entirety.

A single VH or VL domain may be sufficient to provide antigen-binding specificity.

The terms "complementarity determining region" and "CDR" herein are used interchangeably and generally refer to hypervariable regions (HVRs) found in both the light and heavy chain variable domains. The highly conserved portions of the variable domains are called framework regions (FRs). As is known in the art, the amino acid positions representing the hypervariable regions of an antibody may vary according to the context and various definitions known in the art. Some positions within a variable domain may be considered to be hybrid hypervariable positions in that such positions may be considered to be within a hypervariable region according to one set of standards (e.g., IMGT, Chothia, or KABAT) but outside a hypervariable region according to a different set of standards (e.g., KABAT, Chothia, or IMGT). One or more of such positions may also be found in extended hypervariable regions. The present disclosure includes antibodies comprising modifications in such hybrid hypervariable positions. The heavy chain variable region CDR may be abbreviated as HCDR, and the light chain variable region CDR may be abbreviated as LCDR. The variable domains of native heavy and light chains each comprise four framework regions, mainly in a sheet configuration, connected by three CDRs (CDR1, CDR2, and CDR3) that form loops connecting the sheet structures, and in some cases form part of the sheet structures. The CDRs in each chain are held tightly together by the FR regions, in the order of FR1-CDR1-FR2-CDR2-FR3-CDR3-FR4, and contribute to the formation of the antigen-binding site of the antibody with CDRs from other antibody chains (see Kabat et al., Sequences of Proteins of Immunological Interest, National Institute of Health, Bethesda, Md. 1987, the content of which is incorporated herein in its entirety).

For further description of the CDRs, see Kabat et al., J. Biol. Chem., 252: 6609-6616 (1977); Kabat et al., United States Department of Health and Human Services, Sequences of proteins of immunological interest (1991); Chothia et al., J. Mol. Biol., 196: 901-917 (1987); Al-Lazikani B. et al., J. Mol. Biol., 273: 927-948 (1997); MacCallum et al., J. Mol. Biol., 262: 732-745 (1996); Abhinandan and Martin, Mol. Immunol., 45: 3832-3839 (2008); Lefranc M.P. et al., Dev. Comp. Immunol., 27: 55-77 (2003); and Honegger and Plückthun, J. Mol. Biol., 309: 657-670 (2001). "CDR" herein may be labeled and defined in a manner well known in the art, including but not limited to the Kabat numbering scheme, the Chothia numbering scheme, or the IMGT numbering scheme; the tool websites used include, but are not limited to, the AbRSA site (http://cao.labshare.cn/AbRSA/cdrs.php), abYsis site (www.abysis.org/abysis/sequence_input/key_annotation/key_annotation.cgi), and IMGT site (http://www.imgt.org/3Dstructure-DB/cgi/DomainGapAlign.cgi#results). The CDR herein includes overlaps and subsets of amino acid residues defined in different ways. (The above contents are incorporated herein in their entirety).

The term "Kabat numbering scheme" herein generally refers to the immunoglobulin alignment and numbering scheme proposed by Elvin A. Kabat (see, e.g., Kabat et al., Sequences of Proteins of Immunological Interest, 5th Ed. Public Health Service, National Institutes of Health, Bethesda, Md., 1991).

The term "Chothia numbering scheme" herein generally refers to the immunoglobulin numbering scheme proposed by Chothia et al., which is a classical rule for identifying CDR region boundaries based on the position of structural loop regions (see, e.g., Chothia & Lesk (1987) J. Mol. Biol., 196: 901-917; Chothia et al., (1989) Nature, 342: 878-883).

The term "IMGT numbering scheme" herein generally refers to a numbering scheme based on the international ImMunoGeneTics information system (IMGT) initiated by Lefranc et al. See Lefranc et al., Dev. Comparat. Immunol., 27: 55-77, 2003. (The above contents are incorporated herein in their entirety).

The term "heavy chain constant region" herein refers to the carboxyl-terminal portion of an antibody heavy chain that is not directly involved in the binding of the antibody to an antigen, but exhibits effector functions, such as interaction with an Fc receptor; the heavy chain constant region has a more conserved amino acid sequence relative to the variable domain of the antibody. The "heavy chain constant region" may be selected from a CH1 domain, a hinge region, a CH2 domain, a CH3 domain, or a variant or fragment thereof. The "heavy chain constant region" includes a "full-length heavy chain constant region" having a structure substantially similar to that of a natural antibody constant region and a "heavy chain constant region fragment" including only "a portion of the full-length heavy chain constant region". Illustratively, a typical "full-length antibody heavy chain constant region" consists of the CH1 domain-hinge region-CH2 domain-CH3 domain. When the antibody is IgE, it further comprises a CH4 domain; when the antibody is a heavy chain antibody, it does not comprise a CH1 domain. Illustratively, a typical "heavy chain constant region fragment" may be selected from an Fc domain or a CH3 domain.

The term "light chain constant region" herein refers to the carboxyl-terminal portion of an antibody light chain that is not directly involved in the binding of the antibody to an antigen. The light chain constant region may be selected from a constant κ domain or a constant λ domain.

The term "Fc region" herein is used to define the C-terminal region of an antibody heavy chain containing at least a portion of the constant region. The term includes Fc regions of native sequences and variant Fc regions. Illustratively, the human IgG heavy chain Fc region may extend from Cys226 or Pro230 to the carboxyl-terminus of the heavy chain. However, an antibody produced by a host cell may undergo post-translational cleavage of one or more, particularly one or two, amino acids from the C-terminus of the heavy chain. Therefore, the antibody produced by a host cell via the expression of a specific nucleic acid molecule encoding a full-length heavy chain may include a full-length heavy chain, or may include cleaved variants of the full-length heavy chain. It may be such a situation when the last two C-terminal amino acids of the heavy chain are glycine (G446) and lysine (K447, in accordance with the Kabat EU index). Therefore, the C-terminal lysine (Lys447), or the C-terminal glycine (Gly446) and lysine (Lys447), of the Fc region may or may not be present. Typically, the IgG Fc region comprises IgG CH2 and IgG CH3 domains; optionally, the IgG Fc region may further comprise a complete or partial hinge region, but does not comprise a CH1 domain. The "CH2 domain" of the human IgG Fc region typically extends from an amino acid residue at about position 231 to an amino acid residue at about position 340. In one embodiment, a carbohydrate chain is attached to the CH2 domain. The CH2 domain herein may be a CH2 domain of a native sequence or a variant CH2 domain. The "CH3 domain" comprises the residues in the Fc region at the C-terminus of the CH2 domain (i.e., from an amino acid residue at about position 341 to an amino acid residue at about position 447 of the IgG). The CH3 region herein may be a CH3 domain of a native sequence or a variant CH3 domain (e.g., a CH3 domain having a "protuberance" ("knob") introduced into one strand and a "cavity" ("hole") correspondingly introduced into the other strand; see U.S. Pat. No. 5,821,333, the content of which is incorporated herein in its entirety). As described herein, such variant CH3 domains may be used to promote the heterodimerization of two non-identical antibody heavy chains.

Unless otherwise specified herein, amino acid residues in the Fc region or constant region are numbered according to the EU numbering scheme, also known as the EU index, as described in Kabat et al., Sequences of Proteins of Immunological Interest, 5th Ed., Public Health Service, National Institutes of Health, Bethesda, MD, 1991, the content of which is incorporated herein in its entirety. The term "Fc variant" herein refers to a change in the structure or functionality of an Fc caused by the presence of one or more amino acid substitution, insertion, or deletion mutations at appropriate sites on the Fc. The "inter-Fe variant interaction" refers to the formation of space-filling effects, electrostatic steering, hydrogen bonding, hydrophobic interactions, etc., between mutationally designed Fc variants. The inter-Fe variant interaction contributes to the formation of stable heterodimeric proteins. Preferred mutational designs are those of the "knob-into-hole" format.

Techniques for mutational designs in Fc variants have been widely used in the art to prepare bispecific antibodies or heterodimeric Fc fusion protein forms. Representatives are the "knob-into-hole" format proposed by Cater et al. (Protein Engineering, vol. 9 no.7, pp. 617-621, 1996); the Fc-containing heterodimeric format developed by researchers from Amgen using electrostatic steering (US 20100286374 A1); the heterodimeric format (SEEDbodies) formed via IgG/Ig chain exchange proposed by Jonathan H. Davis et al. (Protein Engineering, Design & Selection, pp. 1-8, 2010); the bispecific molecule generated using Genmab's DuoBody platform technology (Science, 2007, 317(5844)); the heterodimeric protein format developed by researchers from Xencor through a combination of structural computation and Fc amino acid mutations across different modes of action (mAbs 3:6, 546-557; November/December 2011); the heterodimeric protein format produced by Alphamab Oncology, Suzhou, via the charge network-based Fc engineering method (CN201110459100.7); and other genetic engineering methods based on Fc amino acid changes or functional modifications to achieve the formation of functional heterodimeric proteins. The knob/hole structures on the Fc variant fragment described in the present disclosure mean that the two Fc fragments are mutated separately and can be bound by the "knob-into-hole" format after mutation. The "knob-into-hole" model of Cater et al. is preferred for site-directed mutagenesis on the Fc region, such that the resulting first and second Fc variants can bind together in the "knob-into-hole" form to give a heterodimer. The selection of a particular immunoglobulin Fc region from a particular immunoglobulin class and subclass is within the knowledge of those skilled in the art. Preferred are the Fc regions of human IgG1, IgG2, IgG3, and IgG4 antibodies, and more preferred is the Fc region of human IgG1 antibody. One of the first Fc variant or the second Fc variant is randomly selected for the knob mutation and the other for the hole mutation. (The above contents are incorporated herein in their entirety).

The term "conservative amino acid" herein generally refers to amino acids that belong to the same class or have similar characteristics (e.g., charge, side chain size, hydrophobicity, hydrophilicity, backbone conformation, and rigidity). Illustratively, the amino acids in each of the following groups belong to conservative amino acid residues of each other, and substitutions of amino acid residues within the groups belong to conservative amino acid substitutions:
1) alanine (A), serine (S), and threonine (T);
2) aspartate (D) and glutamate (E);
3) asparagine (N) and glutamine (Q);
4) arginine (R), lysine (K), and histidine (H);
5) isoleucine (I), leucine (L), methionine (M), and valine (V); and
6) phenylalanine (F), tyrosine (Y), and tryptophan (W).

The term "identity" herein can be obtained by calculating as follows: to determine the percent "identity" of two amino acid sequences or two nucleic acid sequences, the sequences are aligned for optimal comparison purposes (e.g., for optimal alignment, gaps can be introduced in one or both of the first and second amino acid sequences or nucleic acid sequences, or non-homologous sequences can be discarded for comparison purpose). Amino acid residues or nucleotides at corresponding amino acid positions or nucleotide positions are then compared. When a position in the first sequence is occupied by the same amino acid residue or nucleotide at the corresponding position in the second sequence, the molecules are identical at this position. The percent identity between two sequences varies with the identical positions shared by the sequences, taking into account the number of gaps that need to be introduced and the length of each gap for optimal alignment of the two sequences.

A mathematical algorithm can be used to compare two sequences and calculate the percent identity between the sequences. For example, the percent identity between two amino acid sequences is determined with the Needlema and Wunsch algorithm ((1970) J. Mol. Biol., 48: 444-453; available at www.gcg.com) that has been integrated into the GAP program of the GCG software package, using the Blossum 62 matrix or PAM250 matrix and gap weight of 16, 14, 12, 10, 8, 6, or 4 and length weight of 1, 2, 3, 4, 5, or 6. For another example, the percent identity between two nucleotide sequences is determined with the GAP program of the GCG software package (available at www.gcg.com), using the NWSgapdna.CMP matrix and gap weight of 40, 50, 60, 70, or 80 and length weight of 1, 2, 3, 4, 5, or 6. A particularly preferred parameter set (and one that should be used unless otherwise stated) is a Blossum62 scoring matrix with a gap penalty of 12, a gap extension penalty of 4, and a frameshift gap penalty of 5. The percent identity between two amino acid sequences or nucleotide sequences can also be determined with a PAM120 weighted remainder table, a gap length penalty of 12, and a gap penalty of 4, using the E. Meyers and W. Miller algorithm ((1989) CABIOS, 4: 11-17) that has been incorporated into the ALIGN program (version 2.0). (The above contents are incorporated herein in their entirety).

Additionally or alternatively, the nucleic acid sequences and protein sequences described in the present disclosure can be further used as "query sequences" to perform searches against public databases to, e.g., identify other family member sequences or related sequences. For example, such searches can be performed using the NBLAST and XBLAST programs (version 2.0) of Altschul et al., (1990) J. Mol. Biol., 215: 403-10. BLAST nucleotide searches can be performed with the NBLAST program, with a score of 100 and a word length of 12, to obtain nucleotide sequences homologous to the nucleic acid molecule of the present disclosure. BLAST protein searches can be performed using the XBLAST program, with a score of 50 and a word length of 3, to obtain amino acid sequences homologous to the protein molecule of the present disclosure. To obtain gapped alignment results for the purpose of comparison, gapped BLAST can be used as described in Altschul et al., (1997) Nucleic Acids Res., 25: 3389-3402. When using the BLAST and gapped BLAST programs, the default parameters of the respective programs (e.g., XBLAST and NBLAST) can be used. See www.ncbi.nlm.nih.gov. (The above contents are incorporated herein in their entirety).

The term "chimeric antigen receptor (CAR)" herein refers to an artificial cell surface receptor engineered to be expressed on an immune effector cell and to specifically bind to an antigen, which comprises at least (1) an extracellular antigen-binding domain, e.g., a heavy chain variable region and/or a light chain variable region of an antibody, (2) a transmembrane domain that anchors the CAR into the immune effector cell, and (3) an intracellular signaling domain. The CAR is capable of redirecting T cells and other immune effector cells to a selected target, e.g., a cancer cell, in a non-MHC-restricted manner using the extracellular antigen-binding domain.

The term "nucleic acid" herein includes any compound and/or substance that comprises a polymer of nucleotides. Each nucleotide consists of a base, in particular a purine or pyrimidine base (i.e., cytosine (C), guanine (G), adenine (A), thymine (T), or uracil (U)), a sugar (i.e., deoxyribose or ribose), and a phosphate group. Generally, a nucleic acid molecule is described as a sequence of bases, whereby the bases represent the primary structure (linear structure) of the nucleic acid molecule. The sequence of bases is generally expressed as 5' to 3'. Herein, the term "nucleic acid molecule" encompasses deoxyribonucleic acid (DNA), including, e.g., complementary DNA (cDNA) and genomic DNA; ribonucleic acid (RNA), in particular messenger RNA (mRNA); the synthetic forms of DNA or RNA; and polymers comprising a mixture of two or more of these molecules. The nucleic acid molecule may be linear or cyclic. Furthermore, the term "nucleic acid molecule" includes both sense and antisense strands, as well as single- and double-stranded forms. Moreover, the nucleic acid molecules described herein may contain naturally occurring or non-naturally occurring nucleotides. Examples of non-naturally occurring nucleotides include modified nucleotide bases having derived sugar or phosphate backbone bonding or chemically modified residues. The nucleic acid molecule also encompasses DNA and RNA molecules suitable for use as vectors for direct expression of the antibodies of the present disclosure *in vitro* and/or *in vivo,* e.g., in a host or patient. Such DNA (e.g., cDNA) or RNA (e.g., mRNA) vectors may be unmodified or modified. For example, mRNA may be chemically modified to enhance the stability of the RNA vector and/or the expression of the encoded molecule, so that the mRNA can be injected into a subject to produce antibodies *in vivo* (see, e.g., Stadler et al., Nature Medicine, 2017, published online, June 12, 2017, doi: 10.1038/nm.4356 or EP2101823B1, the content of which is incorporated herein in its entirety).

The "isolated" nucleic acid herein refers to a nucleic acid molecule that has been separated from components of its natural environment. The isolated nucleic acid includes a nucleic acid molecule contained in such a cell: the cell generally contains the nucleic acid molecule, but the nucleic acid molecule is present extrachromosomally or at a chromosomal location different from its natural chromosomal location.

The term "vector" herein refers to a nucleic acid molecule capable of amplifying another nucleic acid to which it has been linked. The term includes vectors that serve as self-replicating nucleic acid structures, as well as vectors integrated into the genome of a host cell into which they have been introduced. Certain vectors are capable of directing the expression of nucleic acids to which they are operably linked. Such vectors are called "expression vectors" herein.

The term "host cell" herein refers to a cell into which an exogenous nucleic acid has been introduced, including the progeny of such a cell. Host cells include "transformants" and "transformed cells", which include primary transformed cells and progeny derived therefrom, regardless of the number of passages. Progeny may not be completely identical to parent cells in terms of nucleic acid content, and may contain mutations. Mutant progeny that have the same function or biological activity as the cells screened or selected from the initially transformed cells are included herein.

The term "pharmaceutical composition" herein refers to a formulation that exists in a form allowing the biological activity of the active ingredient contained therein to be effective and does not contain additional ingredients having unacceptable toxicity to a subject to which the pharmaceutical composition is administered.

The term "pharmaceutically acceptable carrier" herein includes any and all solvents, dispersion media, coatings, surfactants, antioxidants, preservatives (e.g., antibacterial agents and antifungal agents), isotonic agents, absorption retardants, salts, preservatives, drug stabilizers, binders, excipients, disintegrants, lubricants, sweeteners, flavorants, dyes, and the like, and combinations thereof, as known to those skilled in the art (see. e.g., Remington's Pharmaceutical Sciences, 18th Ed. MackPrinting Company, 1990, pp. 1289-1329, the content of which is incorporated herein in its entirety). Except insofar as any conventional carrier is incompatible with the active ingredient, its use in the therapeutic or pharmaceutical compositions is contemplated.

The term "treatment" herein refers to surgical or therapeutic treatment for the purpose of preventing or slowing (reducing) an undesired physiological or pathological change, e.g., cancer or tumor, in a subject being treated. Beneficial or desired clinical results include, but are not limited to, alleviation of symptoms, decrease of severity of disease, stabilization (i.e., not worsening) of state of disease, delay or slowing of disease progression, amelioration or palliation of state of disease, and remission of state of disease (whether partial or total), whether detectable or undetectable. Subjects in need of treatment include those already with a disorder or disease, as well as those who are susceptible to a disorder or disease or those who intend to prevent a disorder or disease. When referring to terms such as slowing, alleviation, decrease, palliation, and remission, their meanings also include elimination, disappearance, nonoccurrence, etc.

The term "subject" herein refers to an organism that receives the treatment for a particular disease or disorder described herein. Illustratively, the "subject" includes mammals, such as humans, primates (e.g., monkeys), or non-primate mammals, that receive the treatment for a disease or disorder.

The term "effective amount" herein refers to an amount of a therapeutic agent that is effective to prevent or alleviate symptoms of a disease or the progression of the disease when administered to a cell, tissue, or subject alone or in combination with another therapeutic agent. The "effective amount" also refers to an amount of a compound that is sufficient to alleviate symptoms, e.g., to treat, cure, prevent, or alleviate related medical disorders, or to increase the rates at which such disorders are treated, cured, prevented, or alleviated. When the active ingredient is administered alone to an individual, a therapeutically effective dose refers to the amount of the ingredient alone. When a combination is used, a therapeutically effective dose refers to the combined amounts of the active ingredients that produce the therapeutic effect, whether administered in combination, sequentially, or simultaneously.

The term "cancer" herein refers to or describes a physiological condition in mammals that is typically characterized by unregulated cell growth. Included in this definition are benign and malignant cancers. The term "tumor" or "neoplasm" herein refers to all neoplastic cell growth and proliferation, whether malignant or benign, and all pre-cancerous and cancerous cells and tissues. The terms "cancer" and "tumor" are not mutually exclusive when referred to herein.

### BRIEF DESCRIPTION OF THE DRAWINGS

FIG. 1A shows the binding activity of SCR-10437 to a human B7H3(4Ig)-his protein as assayed by ELISA;
FIG. 1B shows the binding activity of SCR-10437 to a human B7H3(2Ig)-his protein as assayed by ELISA;
FIG. 1C shows the binding activity of SCR-10437 to a monkey B7H3-his protein as assayed by ELISA;
FIGs. 2A-2D show the expression levels of B7H3 in tumor cells A375, A549, 786-O, and NCI-H1975;
FIG. 3A shows the binding reaction of SCR-10437 with a human endogenous tumor cell line A375 as assayed by FACS;
FIG. 3B shows the binding reaction of SCR-10437 with a human endogenous tumor cell line A549 as assayed by FACS;
FIG. 4A shows the endocytic activity of B7H3 antibodies on a tumor cell line 786-O as assayed by FACS;
FIG. 4B shows the endocytic activity of B7H3 antibodies on a tumor cell line NCI-H1975 as assayed by FACS.

### DETAILED DESCRIPTION

The present disclosure will be further described with reference to specific examples, and the advantages and features of the present disclosure will become more apparent with the description.

Experimental procedures without specified conditions in the examples are conducted according to conventional conditions or conditions recommended by the manufacturers. Reagents or instruments without specified manufacturers used herein are conventional products that are commercially available.

The examples of the present disclosure are exemplary only and do not limit the scope of the present disclosure in any way. It will be appreciated by those skilled in the art that various changes or substitutions in forms and details may be made to the technical solutions of the present disclosure without departing from the spirit and scope of the present disclosure, and that these changes and substitutions shall fall within the protection scope of the present disclosure.

### Example 1: Preparation of Recombinant Proteins and Control Antibodies and Purification Method Thereof

### 1.1 Design and expression of recombinant proteins

Construction of B7H3-4Ig recombinant protein: A human B7H3-4Ig protein (UniProt No.: Q5ZPR3-1) was used as a template sequence to design a tagged fusion protein, which was cloned into a pTT5 vector (Youbio, VT2202) to construct a B7H3-4Ig plasmid. Similar construction of B7H3-2Ig recombinant protein: A human B7H3-2Ig protein (UniProt No.: Q5ZPR3-2) was used as a template sequence to design a tagged fusion protein, which was cloned into a pTT5 vector to construct a B7H3-2Ig plasmid. In addition, Expi 293F cells (Gibco, A14527) were transiently transfected to express the antigens and proteins for detection of the present disclosure. The method for preparing the cynomolgus monkey B7H3 recombinant protein was the same as the method for preparing the human recombinant protein. The cynomolgus monkey B7H3 sequence was from Uniprot No.: A0A2K5U2B3. The specific sequence information of the recombinant proteins is shown in Table 1. Purification of recombinant protein by nickel column: The cell expression supernatant sample was centrifuged at high speed to remove impurities, and a nickel column was equilibrated with 20 mM PBS + 500 mM NaCl solution and washed with 2-5 column volumes. The culture supernatant was loaded onto a Ni affinity chromatographic column (purchased from GE Healthcare), and meanwhile, the changes in ultraviolet absorption value (A280 nm) were monitored using an ultraviolet detector. The column was washed with an equilibration buffer until the A280 reading dropped to the baseline, then gradient elution was separately performed using equilibration buffers containing 10 mM, 20 mM, 40 mM, 90 mM, 250 mM, and 500 mM imidazole, and each of the elution peaks was collected. The component of the target protein was determined according to the SDS-PAGE gel image. The eluted product comprising the target protein was collected and concentrated, which may be further purified by gel chromatography Superdex200 (GE) with PBS as the mobile phase to remove aggregates and impurity protein peaks, and the elution peak of the target product was collected. After it was confirmed by electrophoresis, peptide mapping, and LC-MS that the obtained protein was the desired protein, the protein was aliquoted for later use. The proteins obtained by purification according to this protocol include human B7H3 4Ig-His, human B7H3 2Ig-His, and monkey B7H3-His.

**Table 1. Sequences of human and monkey B7H3-His recombinant proteins**

| Antigen name | Sequence No. | Sequence information |
|---|---|---|
| Human B7H3 4Ig-his | SEQ ID NO.1 | his-tagged human B7H3 protein extracellular region fusion protein: |
| | | GALEVQVPEDPVVALVGTDATLCCSFSPEPGFSLAQLNLIWQLTDTK QLVHSFAEGQDQGSAYANRTALFPDLLAQGNASLRLQRVRVADEGS FTCFVSIRDFGSAAVSLQVAAPYSKPSMTLEPNKDLRPGDTVTITCSS YQGYPEAEVFWQDGQGVPLTGNVTTSQMANEQGLFDVHSILRVVL GANGTYSCLVRNPVLQQDAHSSVTITPQRSPTGAVEVQVPEDPVVA LVGTDATLRCSFSPEPGFSLAQLNLIWQLTDTKQLVHSFTEGRDQGS AYANRTALFPDLLAQGNASLRLQRVRVADEGSFTCFVSIRDFGSAAV SLQVAAPYSKPSMTLEPNKDLRPGDTVTITCSSYRGYPEAEVFWQD GQGVPLTGNVTTSQMANEQGLFDVHSVLRVVLGANGTYSCLVRNP VLQQDAHGSVTITGQPMTGGGSGGGSHHHHHHHHHH |
| Human B7H3 2Ig-his | SEQ ID NO.2 | his-tagged human B7H3 protein extracellular region fusion protein: |
| | | LEVQVPEDPVVALVGTDATLCCSFSPEPGFSLAQLNLIWQLTDTKQL VHSFAEGQDQGSAYANRTALFPDLLAQGNASLRLQRVRVADEGSFT CFVSIRDFGSAAVSLQVAAPYSKPSMTLEPNKDLRPGDTVTITCSSYR GYPEAEVFWQDGQGVPLTGNVTTSQMANEQGLFDVHSVLRVVLG ANGTYSCLVRNPVLQQDAHGSVTITGQPMTFPGGGSGGGSHHHHH HHHHH |
| Monkey B7H3-his | SEQ ID NO.3 | his-tagged cynomolgus monkey B7H3 protein extracellular region fusion protein: |
| | | LEVQVPEDPVVALVGTDATLRCSFSPEPGFSLAQLNLIWQLTDTKQL VHSFTEGRDQGSAYANRTALFLDLLAQGNASLRLQRVRVADEGSFT CFVSIRDFGSAAVSLQVAAPYSKPSMTLEPNKDLRPGDTVTITCSSYR GYPEAEVFWQDGQGAPLTGNVTTSQMANEQGLFDVHSVLRVVLG ANGTYSCLVRNPVLQQDAHGSITITPQRSPTGAVEVQVPEDPVVALV GTDATLRCSFSPEPGFSLAQLNLIWQLTDTKQLVHSFTEGRDQGSAY ANRTALFLDLLAQGNASLRLQRVRVADEGSFTCFVSIRDFGSAAVSL QVAAPYSKPSMTLEPNKDLRPGDTVTITCSSYRGYPEAEVFWQDGQ GAPLTGNVTTSQMANEQGLFDVHSVLRVVLGANGTYSCLVRNPVL QQDAHGSVTITGQPMTFPPEGGGSGGGSHHHHHHHHHH |

### 1.2 Design and expression of control antibodies

The control antibodies used in the present disclosure were all derived from published patents. The DS7300 antibody was derived from the published patent CN103687945B, MGC018 was derived from the published patent WO2017180813A, and the Enoblituzumab antibody was derived from the published patent WO2011109400A2. Unless otherwise stated, the DS7300, Enoblituzumab, and MGC018 control antibodies were all recombinantly expressed using the human IgG1 + κ subtype.

The expression and purification processes of the control antibody were as follows: The antibody sequence gene was synthesized and cloned into an expression vector pTT5, Expi293F cells (purchased from Gibco, A14527) were then transiently transfected, and after the cells were cultured on a shaker at 37 °C for 7 days, the cell supernatant was collected for Protein A antibody purification. The Protein A affinity column was washed with 3-5 column volumes of 0.1 M NaOH, then with 3-5 column volumes of pure water. The chromatographic column was equilibrated with 3-5 column volumes of a 1× PBS (pH 7.4) buffer system as an equilibration buffer. The cell supernatant was loaded onto the column at a low flow rate for binding, with the flow rate controlled to allow for a retention time of about 1 min or longer. After the binding was completed, the chromatographic column was washed with 3-5 column volumes of 1× PBS (pH 7.4) until the ultraviolet absorbance fell back to baseline. The sample was eluted with a 50 mM citric acid/sodium citrate (pH 3.0-3.5) buffer, the elution peaks were collected by ultraviolet detection, and the eluted product was rapidly adjusted to pH 5-6 with 1 M Tris-HCl (pH 8.0) and temporarily stored. For the eluted product, solution exchange may be performed by methods well known to those skilled in the art, such as ultrafiltration concentration using an ultrafiltration tube and exchange of the solution to a desired buffer system, or exchange with a desired buffer system by molecular exclusion (e.g., G-25 desalination), or removal of aggregate components from the eluted product using a high-resolution molecular exclusion column such as Superdex 200 to improve sample purity. The resulting control antibodies were named DS7300-hIgG1, MGC018-hIgG1, and Enoblituzumab-hIgG1. The specific sequence information of the antibodies is shown in Table 2.

**Table 2. Sequence listing of control antibodies**

| Antibody name | Sequence No. | Sequence |
|---|---|---|
| DS7300-hIgG1 (heavy chain) | SEQ ID NO.4 | QVQLVQSGAEVKKPGSSVKVSCKASGYTFTNYVMHWVRQAPGQGL EWMGYINPYNDDVKYNEKFKGRVTITADESTSTAYMELSSLRSEDTAV YYCARWGYYGSPLYYFDYWGQGTLVTVSSASTKGPSVFPLAPSSKST SGGTAALGCLVKDYFPEPVTVSWNSGALTSGVHTFPAVLQSSGLYSLS SVVTVPSSSLGTQTYICNVNHKPSNTKVDKKVEPKSCDKTHTCPPCPA PELLGGPSVFLFPPKPKDTLMISRTPEVTCVVVDVSHEDPEVKFNWYV DGVEVHNAKTKPREEQYNSTYRVVSVLTVLHQDWLNGKEYKCKVSN KALPAPIEKTISKAKGQPREPQVYTLPPSREEMTKNQVSLTCLVKGFYP SDIAVEWESNGQPENNYKTTPPVLDSDGSFFLYSKLTVDKSRWQQGN VFSCSVMHEALHNHYTQKSLSLSPGK |
| DS7300-hIgK (light chain) | SEQ ID NO.5 | EIVLTQSPATLSLSPGERATLSCRASSRLIYMHWYQQKPGQAPRPLIYAT SNLASGIPARFSGSGSGTDFTLTISSLEPEDFAVYYCQQWNSNPPTFGQ GTKVEIKRTVAAPSVFIFPPSDEQLKSGTASVVCLLNNFYPREAKVQW KVDNALQSGNSQESVTEQDSKDSTYSLSSTLTLSKADYEKHKVYACE VTHQGLSSPVTKSFNRGEC |
| MGC018-hIgG1 (heavy chain) | SEQ ID NO.6 | EVQLVESGGGLVKPGGSLRLSCAASGFTFSSYGMSWVRQAPGKGLEW VATINSGGSNTYYPDSLKGRFTISRDNAKNSLYLQMNSLRAEDTAVYY CARHDGGAMDYWGQGTTVTVSSASTKGPSVFPLAPSSKSTSGGTAAL GCLVKDYFPEPVTVSWNSGALTSGVHTFPAVLQSSGLYSLSSVVTVPSS SLGTQTYICNVNHKPSNTKVDKKVEPKSCDKTHTCPPCPAPELLGGPS VFLFPPKPKDTLMISRTPEVTCVVVDVSHEDPEVKFNWYVDGVEVHN AKTKPREEQYNSTYRVVSVLTVLHQDWLNGKEYKCKVSNKALPAPIE KTISKAKGQPREPQVYTLPPSREEMTKNQVSLTCLVKGFYPSDIAVEW ESNGQPENNYKTTPPVLDSDGSFFLYSKLTVDKSRWQQGNVFSCSVM HEALHNHYTQKSLSLSPGK |
| MGC018-hIgK (light chain) | SEQ ID NO.7 | DIQMTQSPSSLSASVGDRVTITCRASESIYSYLAWYQQKPGKAPKLLV |
| | | YNTKTLPEGVPSRFSGSGSGTDFTLTISSLQPEDFATYYCQHHYGTPPW TFGQGTRLEIKRTVAAPSVFIFPPSDEQLKSGTASVVCLLNNFYPREAK VQWKVDNALQSGNSQESVTEQDSKDSTYSLSSTLTLSKADYEKHKVY ACEVTHQGLSSPVTKSFNRGEC |
| Enoblituzumab-hIgG1 (heavy chain) | SEQ ID NO.8 | EVQLVESGGGLVQPGGSLRLSCAASGFTFSSFGMHWVRQAPGKGLEW VAYISSDSSAIYYADTVKGRFTISRDNAKNSLYLQMNSLRDEDTAVYY CGRGRENIYYGSRLDYWGQGTTVTVSSASTKGPSVFPLAPSSKSTSGG TAALGCLVKDYFPEPVTVSWNSGALTSGVHTFPAVLQSSGLYSLSSVV TVPSSSLGTQTYICNVNHKPSNTKVDKKVEPKSCDKTHTCPPCPAPEL LGGPSVFLFPPKPKDTLMISRTPEVTCVVVDVSHEDPEVKFNWYVDG VEVHNAKTKPREEQYNSTYRVVSVLTVLHQDWLNGKEYKCKVSNKA LPAPIEKTISKAKGQPREPQVYTLPPSREEMTKNQVSLTCLVKGFYPSDI AVEWESNGQPENNYKTTPPVLDSDGSFFLYSKLTVDKSRWQQGNVFS CSVMHEALHNHYTQKSLSLSPGK |
| Enoblituzumab-hIgK (light chain) | SEQ ID NO.9 | DIQLTQSPSFLSASVGDRVTITCKASQNVDTNVAWYQQKPGKAPKALI YSASYRYSGVPSRFSGSGSGTDFTLTISSLQPEDFATYYCQQYNNYPFT FGQGTKLEIKRTVAAPSVFIFPPSDEQLKSGTASVVCLLNNFYPREAKV QWKVDNALQSGNSQESVTEQDSKDSTYSLSSTLTLSKADYEKHKVYA CEVTHQGLSSPVTKSFNRGEC |
| Human hIgG1 heavy chain constant region sequence (HC) | SEQ ID NO.10 | ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK RVEPKSCDKTHTCPPCPAPELLGGPSVFLFPPKPKDTLMISRTPEVTCV VVDVSHEDPEVKFNWYVDGVEVHNAKTKPREEQYNSTYRVVSVLTV LHQDWLNGKEYKCKVSNKALPAPIEKTISKAKGQPREPQVYTLPPSRE EMTKNQVSLTCLVKGFYPSDIAVEWESNGQPENNYKTTPPVLDSDGSF FLYSKLTVDKSRWQQGNVFSCSVMHEALHNHYTQKSLSLSPGK |
| Human κ chain constant region sequence (LC) | SEQ ID NO.11 | |

### Example 2: Preparation of Antibody against B7H3

### 2.1 Animal immunization

The monoclonal antibody of the present disclosure was produced by immunization of mice. The experimental mice were female mice aged 6-8 weeks. The immunogen was the human B7H3 4Ig-his protein prepared in Example 1. During the protein primary immunization, the immunogen was emulsified with TiterMax (purchased from Sigma, T2684-1M) and then injected subcutaneously (SC) and intraperitoneally (IP) at 0.1 mL each; that is, 50 µg of the immunogen was injected into each mouse; during the booster immunization, the immunogen was injected subcutaneously and intraperitoneally with Imject Alum (purchased from Thermo) at 0.1 mL each; that is, 25 µg of the immunogen was injected into each mouse. The frequency of immunization was once a week, and blood samples were collected on day 3, day 19, day 47, and day 61. The presence of antibodies recognizing human B7H3 and the antibody titer in serum of immunized animals were determined by detecting the binding to the human B7H3 protein (His tag, coating concentration: 2 µg/mL) by an ELISA method. According to the serum titer, mice in which the antibody titer in serum was high and tended to plateau were selected for booster immunization.

### 2.2 Antibody screening and sequencing

After completion of the booster immunization, antigen-specific B cells were directly isolated from the immunized mice without fusion with myeloma cells. Plasma cells secreting antigen-specific monoclonal antibodies were screened by using single-cell technology (Beacon^{®} Optofluidic System, Berkeley Lights Inc). The sequences of the light and heavy chain variable regions of the antibody were directly obtained from antigen-specific B cells by reverse transcription and PCR sequencing. The humanized anti-B7H3 antibody obtained ultimately by screening using a conventional method was named SCR10437. The amino acid sequences of the heavy chain variable region (HCVR) and light chain variable region (LCVR) are shown below:
SCR10437 HCVR (SEQ ID NO. 12): 
QVQLVQSGGGVVQPGRSLRLSCAASGFTFSSYGMHWVRQAPGKGLEWVAVIWYAGSNKY
YADSVKGRFTISRDNSKNTVYLQMNSLRAEDTAVYYCARDPYGNNYYYYYYGMDVWGQ
GTTVTVSS.
SCR10437 LCVR (SEQ ID NO. 13):

EIVLTQSPSFLSASVGDRVTITCRASQGISSYLAWYQQKPGKAPKLLIYAASTLQSGVPSRFS
GSGSETEFTLTISSLQPEDFATYYCQQLNSYPPTFGQGTKLEIK.

The CDR regions of the B7H3 monoclonal antibody described above were analyzed, where the CDR regions were determined and annotated using the Kabat numbering scheme, the Chothia numbering scheme, and the IMGT numbering scheme. The specific results are shown in Table 3.

**Table 3. Antibody CDR sequences and numbers**

| Name | Kabat | | IMGT | | Chothia | |
|---|---|---|---|---|---|---|
| SCR1043 7_HCVR | HCDR1 | SYGMH SEQ ID NO.14 | HCDR1 | GFTFSSYG SEQ ID NO.20 | HCDR1 | GFTFSSY SEQ ID NO.26 |
| | HCDR2 | VIWYAGSNKYYADS VKG SEQ ID NO.15 | HCDR2 | IWYAGSNK SEQ ID NO.21 | HCDR2 | WYAGSN SEQ ID NO.27 |
| | HCDR3 | DPYGNNYYYYYYG MDV SEQ ID NO.16 | HCDR3 | ARDPYGNNYY YYYYGMDV SEQ ID NO.22 | HCDR3 | DPYGNNYYYYYY GMDV SEQ ID NO.28 |
| SCR1043 7_LCVR | LCDR1 | RASQGISSYLA SEQ ID NO.17 | LCDR1 | QGISSY SEQ ID NO.23 | LCDR1 | RASQGISSYLA SEQ ID NO.29 |
| | LCDR2 | AASTLQS SEQ ID NO.18 | LCDR2 | AAS SEQ ID NO.24 | LCDR2 | AASTLQS SEQ ID NO.30 |
| | LCDR3 | QQLNSYPPT SEQ ID NO.19 | LCDR3 | QQLNSYPPT SEQ ID NO.25 | LCDR3 | QQLNSYPPT SEQ ID NO.31 |

### 2.3 Construction, expression, and purification of fully human-derived antibody

Primers were designed according to the sequencing results of the variable region genes of the SCR10437 antibody obtained in section 2.2 above, and VH/VL gene fragments of the antibody were constructed by PCR. The obtained VH/VL gene fragments were subjected to homologous recombination with an expression vector pTT5 to construct a full-length expression vector plasmid for the fully human-derived antibody. The SCR10437 antibody was expressed in a human IgG1 format (the sequences of the heavy chain constant region and the light chain constant region are shown in Table 2). After completion of the plasmid preparation, Expi293F cells were transfected and cultured on a shaker at 37 °C for 7 days. The supernatant was then collected and centrifuged, and the antibody was purified by the purification method described in section 1.2 of Example 1. The resulting antibody was named SCR10437-hIgG1.

### Example 3: Assay on Affinity of Antibodies

The strength of antibody-antigen binding was measured on a BIAcore 8K system using the Protein A capture method. First, Protein A was immobilized onto a CM4 chip (purchased from GE, BR-1005-34) using an amino coupling method. According to the instruction of the Amine Coupling Kit (purchased from GE, BR100633), NHS and EDC were mixed, with HBS-EP+ pH 7.4 as a mobile phase, to activate the chip for about 600 s; the Protein A was diluted to 50 µg/mL with 10 mM sodium acetate (pH 4.5) and injected for 600 s; and finally, the remaining activated sites were blocked with ethanolamine. Then, the affinity of the antibody for the antigen was assayed using a multi-cycle kinetic method. In each cycle, first, a test antibody was captured using a Protein A chip, and then a single concentration of the antigen protein was injected. The association and dissociation processes of the antibody with the antigen protein were recorded, and finally, the chip was regenerated using Glycine (pH 1.5), wherein the mobile phase was HBS-EP+ (10 mM HEPES, 150 mM NaCl, 3 mM EDTA, 0.05% surfactant P20), the flow rate was 30 µL/min, the regeneration time was 30 s, and the assay temperature was 25 °C. Finally, according to a 1:1 binding model, the data were analyzed, and the antibody-antigen binding kinetic parameters, including the association rate constant Ka, the dissociation rate constant Kd, the equilibrium dissociation constant KD, and the maximum binding signal Rmax, were fitted.

The association rate (Ka), dissociation rate (Kd), and binding affinity (KD) of the SCR10437-hIgG1 antibody with human and monkey B7H3 proteins are shown in Table 4.

**Table 4. Affinity of antibodies for B7H3 proteins as detected by SPR (biacore)**

| Antigen name | Antibody name | Ka (1/Ms) | Kd (1/s) | KD (M) |
|---|---|---|---|---|
| Human B7H3(4Ig)-his | SCR10437-hIgG1 | 1.07E+05 | 8.49E-05 | 7.96E-10 |
| | DS7300-hIgG1 | 6.65E+05 | 1.22E-04 | 1.84E-10 |
| Human B7H3(2Ig)-his | SCR10437-hIgG1 | 8.77E+04 | 4.64E-04 | 5.29E-09 |
| | DS7300-hIgG1 | 4.82E+05 | 9.72E-03 | 2.02E-08 |
| cyno B7H3-his | SCR10437-hIgG1 | 9.21E+04 | 9.29E-05 | 1.01E-09 |
| | DS7300-hIgG1 | 6.89E+05 | 1.62E-03 | 2.35E-09 |

### Example 4: Identification of Binding Activity of Antibodies

### 4.1 Assay on binding of fully human-derived antibodies to human and monkey B7H3 proteins by ELISA

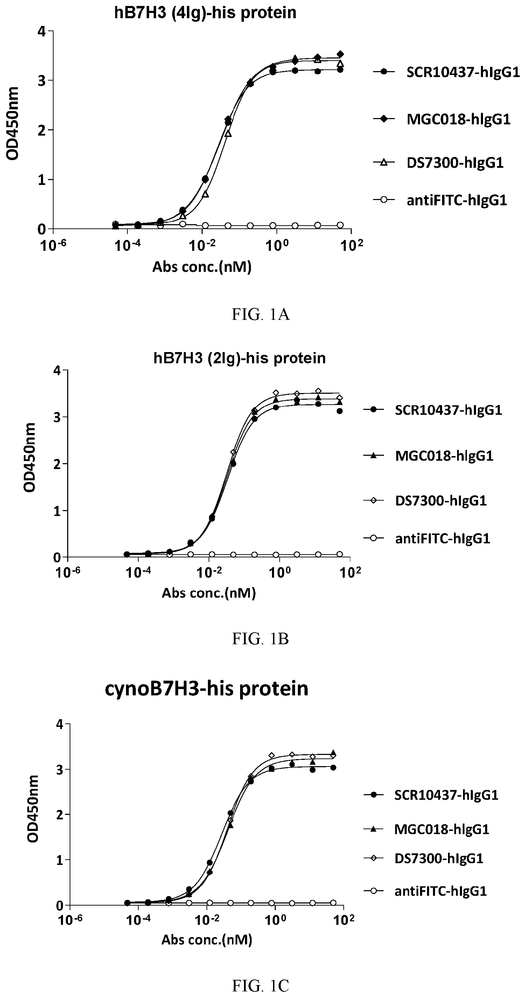

A human B7H3(4Ig)-his protein was diluted with PBS to a final concentration of 1 µg/mL and then added to a 96-well ELISA plate at 50 µL/well, followed by incubation at 4 °C overnight. The next day, the plate was washed twice with PBST, and a blocking buffer [PBS + 2% (w/w) BSA] was added for blocking at room temperature for 2 h. The blocking buffer was discarded, and the antibody and the positive and negative control antibodies (diluted in a 4-fold gradient from an initial concentration of 50 nM) were added at 50 µL/well. After incubation at 37 °C for 1 h, the plate was washed 3 times with PBST. A horseradish peroxidase (HRP)-labeled secondary antibody (purchased from Merck, Cat. No. AP113P) was added. After incubation at 37 °C for 1 h, the plate was washed 5 times with PBST. A TMB substrate was added at 50 µL/well. After incubation at room temperature for 10 min, a stop solution (1.0 M HCl) was added at 50 µL/well. The OD_{450 nm} values were read using an ELISA plate reader (Multimode Plate Reader, EnSight, purchased from Perkin Elmer). The binding activity of the antibodies to the human B7H3(4Ig)-his protein is shown in FIG. 1A. The results show that the negative control anti-FITC-hIgG1 (sourced from J Biol Chem., 1990 Jan. 5; 265(1): 133-8) did not bind to B7H3, the SCR10437-hIgG1 antibody was capable of effectively binding to the human B7H3(4Ig)-his protein, and the binding ability of the SCR10437-hIgG1 antibody was comparable to that of the positive control antibody DS7300-hIgG1.

A human B7H3(2Ig)-his protein was diluted with PBS to a final concentration of 2 µg/mL and then added to a 96-well ELISA plate at 50 µL/well, followed by incubation at 4 °C overnight. The binding activity of the B7H3 antibodies to the human B7H3(2Ig)-his protein was analyzed using the same ELISA assay method as described above. The results show that the SCR10437-hIgG1 antibody was capable of effectively binding to the human B7H3(2Ig)-his protein, and the binding ability of the SCR10437-hIgG1 antibody was comparable to that of the positive control antibody DS7300-hIgG1 (FIG. 1B).

A monkey cynoB7H3-his protein was diluted with PBS to a final concentration of 2 µg/mL and then added to a 96-well ELISA plate at 50 µL/well, followed by incubation at 4 °C overnight. The binding activity of the B7H3 antibodies to the cynoB7H3-his protein was analyzed using the same assay method as described above. The results show that the SCR10437-hIgG1 antibody was capable of effectively binding to the cynoB7H3-his protein, and the binding ability of the SCR10437-hIgG1 antibody was comparable to that of the positive control antibody DS7300-hIgG1 (FIG. 1C).

### 4.2 Assay on binding of B7H3 antibodies to tumor cells by flow cytometry assay (FACS)

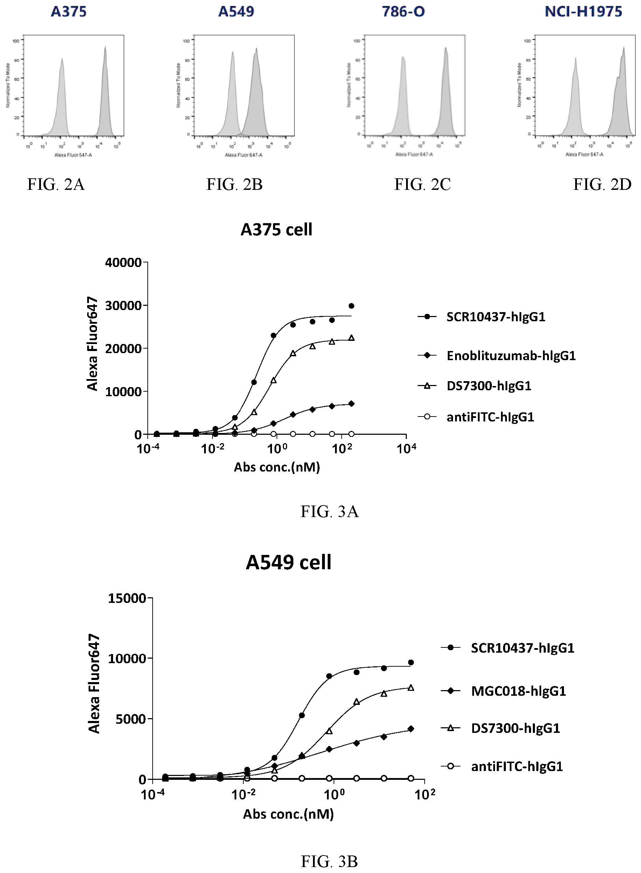

Endogenous tumor cells A375 (Nanjing Cobioer, CBP60329, see FIG. 2A for the expression level of B7H3) were expanded to the logarithmic growth phase in a T-175 culture flask, the medium was removed by pipetting, and the cells were washed twice with a PBS buffer and digested with trypsin. Then, a complete medium was added to stop the digestion, and the cells were pipetted to form a single cell suspension. After counting, the cells were centrifuged, and the cell pellet was resuspended to 2 × 10⁶ cells/mL in an FACS buffer (PBS + 10% fetal bovine serum) and added to a 96-well FACS reaction plate at 100 µL/well. The plate was centrifuged, and the supernatant was discarded. A test antibody sample (diluted in a 4-fold gradient from an initial concentration of 200 nM) was added at 50 µL/well and mixed well with the cells, and the mixture was incubated at 4 °C for 1 h. After the plate was centrifuged and washed 3 times with a PBS buffer, 50 µL of Alexa Fluor^{®} 647 AffiniPure Goat Anti-Human IgG, Fcy fragment specific-labeled secondary antibody (purchased from Jackson, Cat. No. 109-605-098) was added to each well, and the mixture was incubated at 4 °C for 1 h. The results were assayed and analyzed by FACS (FACS CantoTM, purchased from BD) after the plate was centrifuged and washed 3 times with a PBS buffer and the cells were resuspended in 100 µL of PBS. Data analysis was performed by using the software (FlowJo) to obtain the mean fluorescence intensity (MFI) of the cells. Then, analysis was performed by using the software (GraphPad Prism8), data were fitted, and EC50 values were calculated. As shown in FIG. 3A and Table 5, the SCR10437-hIgG1 antibody was capable of specifically binding to A375 cells, and the binding ability of the SCR10437-hIgG1 antibody was stronger than that of DS7300-hIgG1 and Enoblituzumab-hIgG1.

**Table 5. Binding of B7H3 antibodies to A375 cells**

| Antibody | EC50, nM | Fluorescence intensity Top value - MFI |
|---|---|---|
| SCR10437-hIgG1 | 0.2359 | 29860 |
| Enoblituzumab-hlgG1 | 1.680 | 7143 |
| DS7300-hIgG1 | 0.5990 | 22468 |
| antiFITC-hIgG1 | / | 84 |

The same method was used to assay the binding of the SCR10437-hIgG1 antibody to a lung cancer cell line, A549 (Nanjing Cobioer, CBP60084, see FIG. 2B for the expression level of B7H3), with moderate expression of B7H3. The results as shown in FIG. 3B and Table 6 indicate that SCR10437-hIgG1 had good binding activity to A549, and the binding ability of SCR10437-hIgG1 was stronger than that of DS7300-hIgG1 and MGC018-hIgG 1.

**Table 6. Binding of B7H3 antibodies to A549 cells**

| Antibody | EC50, nM | Fluorescence intensity Top value - MFI |
|---|---|---|
| SCR10437-hIgG1 | 0.1645 | 9645 |
| MGC018-hlgG1 | 0.5387 | 4171 |
| DS7300-hIgG1 | 0.6802 | 7578 |
| antiFITC-hIgG1 | / | 84 |

### Example 5: Assay on Endocytic Activity of Antibodies

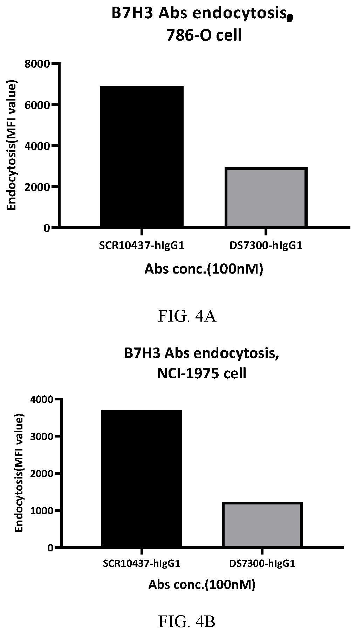

To evaluate the endocytic activity of the anti-B7H3 antibody SCR10437, 786-O (Nanjing Cobioer, CBP60442, see FIG. 2C for the expression level of B7H3) and NCI-H1975 (Nanjing Cobioer, CBP60121, see FIG. 2D for the expression level of B7H3) cells were cultured until they reached 80% confluence. The cells were digested and collected, followed by staining with a test anti-B7H3 antibody (100 nM) and a secondary antibody, Aleax Flour 488-conjugated anti-human IgG, Fcγ fragment specific antibody (2.2 µg/mL, Jackson, 109-545-098) at 4 °C. The cells were washed and incubated at 37 °C for 0 h (pre-incubation) and 4 h. The cells were washed. The pre-incubated or incubated cells were treated with an anti-Alexa488 antibody (Invitrogen, A11094) at a concentration of 10 µg/mL to either quench the fluorescence on the cell surface or leave the fluorescence unquenched to detect the entire signal, and then the cells were analyzed by a flow cytometer. The internalization rate (%) was calculated according to the following formula: [1 - (Na - Qa)/(Na - Na × Qi/Ni)] × 100, and the internalization amount (MFI value) was calculated according to the following formula: Qa - Qi, where Na is the mean fluorescence intensity (MFI) of samples at each incubation time under the unquenched condition, Qa is the MFI of samples at each incubation time under the quenched condition, Ni is the MFI of pre-incubated samples under the unquenched condition, and Qi is the MFI of pre-incubated samples under the quenched condition. The internalization amount represents the total amount of antibody endocytosed into the cell within 4 h. The results as shown in FIG. 4A and FIG. 4B indicate that both the antibodies could be endocytosed, and the endocytosis of SCR10437-hIgG1 was superior to that of the positive control antibody.

## Claims

1. An antibody or an antigen-binding fragment thereof specifically binding to B7H3, comprising a heavy chain variable region (VH) and a light chain variable region (VL), wherein
(1) the heavy chain variable region comprises an HCDR1, an HCDR2, and an HCDR3, wherein the HCDR1, the HCDR2, and the HCDR3 are an HCDR1, an HCDR2, and an HCDR3 of a VH domain set forth in SEQ ID NO. 12; and
(2) the light chain variable region comprises an LCDR1, an LCDR2, and an LCDR3, wherein the LCDR1, the LCDR2, and the LCDR3 are an LCDR1, an LCDR2, and an LCDR3 of a VL domain set forth in SEQ ID NO. 13.

2. The antibody or the antigen-binding fragment thereof according to claim 1, wherein the HCDR1, the HCDR2, and the HCDR3 have the amino acid sequences set forth in SEQ ID NOs. 14-16, SEQ ID NOs. 20-22, or SEQ ID NOs. 26-28 or a sequence combination having 1, 2, 3, or more amino acid insertions, deletions, and/or substitutions compared with the amino acid sequences set forth in SEQ ID NOs. 14-16, SEQ ID NOs. 20-22, or SEQ ID NOs. 26-28, as determined according to the Kabat, Chothia, or IMGT numbering scheme.

3. The antibody or the antigen-binding fragment thereof according to claim 1, wherein the LCDR1, the LCDR2, and the LCDR3 have the amino acid sequences set forth in SEQ ID NOs. 17-19, SEQ ID NOs. 23-25, or SEQ ID NOs. 29-31 or a sequence combination having 1, 2, 3, or more amino acid insertions, deletions, and/or substitutions compared with the amino acid sequences set forth in SEQ ID NOs. 17-19, SEQ ID NOs. 23-25, or SEQ ID NOs. 29-31, as determined according to the Kabat, Chothia, or IMGT numbering scheme.

4. The antibody or the antigen-binding fragment thereof according to any one of claims 1-3, wherein the antibody or the antigen-binding fragment thereof comprises the following LCDR1, LCDR2, LCDR3, HCDR1, HCDR2, and HCDR3:
(1) according to the Kabat numbering scheme, LCDR1-3 and HCDR1-3 having the amino acid sequences set forth in SEQ ID NOs. 14-19 or a sequence combination having 1, 2, 3, or more amino acid insertions, deletions, and/or substitutions compared with the amino acid sequences set forth in SEQ ID NOs. 14-19;
(2) according to the IMGT numbering scheme, LCDR1-3 and HCDR1-3 having the amino acid sequences set forth in SEQ ID NOs. 20-25 or a sequence combination having 1, 2, 3, or more amino acid insertions, deletions, and/or substitutions compared with the amino acid sequences set forth in SEQ ID NOs. 20-25;
(3) according to the Chothia numbering scheme, LCDR1-3 and HCDR1-3 having the amino acid sequences set forth in SEQ ID NOs. 26-31 or a sequence combination having 1, 2, 3, or more amino acid insertions, deletions, and/or substitutions compared with the amino acid sequences set forth in SEQ ID NOs. 26-31.

5. The antibody or the antigen-binding fragment thereof according to any one of claims 1-4, wherein the sequence of the heavy chain variable region comprises the sequence set forth in SEQ ID NO. 12 or a sequence having 80%, 85%, 90%, 95%, 96%, 97%, 98%, 99%, or higher identity thereto; the sequence of the light chain variable region comprises the sequence set forth in SEQ ID NO. 13 or a sequence having 80%, 85%, 90%, 95%, 96%, 97%, 98%, 99%, or higher identity thereto.

6. The antibody or the antigen-binding fragment thereof according to any one of claims 1-5, wherein the antibody or the antigen-binding fragment thereof is fully human-derived.

7. The antibody or the antigen-binding fragment thereof according to any one of claims 1-6, wherein the antibody or the antigen-binding fragment thereof is capable of specifically binding to a human or monkey B7H3 protein.

8. The antibody or the antigen-binding fragment thereof according to any one of claims 1-7, wherein the antibody or the antigen-binding fragment thereof may further comprise any constant region sequence of a human or murine antibody IgG1, IgG2, IgG3, IgG4, IgA, IgM, IgE, or IgD, preferably a constant region sequence of a human or murine antibody IgG1, IgG2, IgG3, or IgG4, or a constant region sequence of a human or murine antibody IgG1, IgG2, IgG3, or IgG4 having a mutation; further, the antibody or the antigen-binding fragment thereof is further conjugated to a therapeutic agent or a tracer; preferably, the therapeutic agent is selected from a radioisotope, a chemotherapeutic agent, a cytotoxic agent, or an immunomodulator, and the tracer is selected from a radiocontrast medium, a paramagnetic ion, a metal, a fluorescent label, a chemiluminescent label, an ultrasound contrast agent, and a photosensitizer; more preferably, the cytotoxic agent is selected from alkaloids, methotrexate, anthracyclines (doxorubicin), pyrrolobenzodiazepine (PBD), gemcitabine, cytarabine, tegafur, ifosfamide, dacarbazine, and oxaliplatin; more preferably, the cytotoxic agent is a taxane.

9. The antibody or the antigen-binding fragment thereof according to any one of claims 1-8, wherein the antigen-binding fragment is selected from one or more of a F(ab')₂, a Fab', a Fab, an Fv, an scFv, a nanobody, or an affibody.

10. A multispecific antigen-binding molecule, comprising the antibody or the antigen-binding fragment thereof according to any one of claims 1-9, wherein preferably, the multispecific antigen-binding molecule further comprises an antibody or an antigen-binding fragment thereof specifically binding to an antigen other than B7H3 or binding to a B7H3 epitope different from a B7H3 epitope to which the antibody or the antigen-binding fragment thereof according to any one of claims 1-9 binds;
preferably, the antigen other than B7H3 is selected from the group consisting of: (1) a tumor-specific antigen (TSA) or a tumor-associated antigen (TAA); (2) an immune checkpoint; and (3) a target for recruiting and/or activating an immune cell;
preferably, the multispecific antigen-binding molecule may be bispecific, trispecific, or tetraspecific; preferably, the multispecific antigen-binding molecule may be divalent, trivalent, tetravalent, pentavalent, or hexavalent.

11. A chimeric antigen receptor (CAR), comprising at least an extracellular antigen-binding domain, a transmembrane domain, and an intracellular signaling domain, wherein the extracellular antigen-binding domain comprises the antibody or the antigen-binding fragment thereof according to any one of claims 1-9.

12. An immune effector cell expressing the chimeric antigen receptor according to claim 11, or comprising a nucleic acid fragment encoding the chimeric antigen receptor according to claim 11, wherein preferably, the immune effector cell is selected from a T cell, a natural killer cell (NK cell), a natural killer T cell (NKT cell), a double negative T cell (DNT cell), a monocyte, a macrophage, a dendritic cell, or a mast cell, and the T cell is selected from a cytotoxic T cell, a regulatory T cell, or a helper T cell; preferably, the immune effector cell is an auto-immune effector cell or an allogeneic immune effector cell.

13. An isolated nucleic acid fragment encoding the antibody or the antigen-binding fragment thereof according to any one of claims 1-9, or the multispecific antigen-binding molecule according to claim 10, or the chimeric antigen receptor according to claim 11.

14. A vector, comprising the nucleic acid fragment according to claim 13.

15. A host cell, comprising the vector according to claim 14, wherein preferably, the cell is a prokaryotic cell or a eukaryotic cell, such as a bacterium (*E. coli*), a fungus (yeast), an insect cell, or a mammalian cell (a CHO cell line or a 293T cell line).

16. A method for preparing the antibody or the antigen-binding fragment thereof according to any one of claims 1-9 or the multispecific antigen-binding molecule according to claim 10, comprising culturing the host cell according to claim 15 and isolating an antibody or an antigen-binding fragment thereof expressed by the host cell, or isolating a multispecific antigen-binding molecule expressed by the host cell.

17. A method for preparing the immune effector cell according to claim 12, comprising introducing a nucleic acid fragment encoding the CAR according to claim 11 into the immune effector cell, wherein optionally, the method further comprises initiating expression of the CAR according to claim 11 by the immune effector cell.

18. A pharmaceutical composition, comprising the antibody or the antigen-binding fragment thereof according to any one of claims 1-9, or the multispecific antigen-binding molecule according to claim 10, or the immune effector cell according to claim 12, or the nucleic acid fragment according to claim 13, or the vector according to claim 14, or the host cell according to claim 15, or an antibody or an antigen-binding fragment thereof or a multispecific antigen-binding molecule prepared by the method according to claim 16, or an immune effector cell prepared by the method according to claim 17, wherein optionally, the pharmaceutical composition further comprises a pharmaceutically acceptable carrier, diluent, or adjuvant.

19. Use of the antibody or the antigen-binding fragment thereof according to any one of claims 1-9, or the multispecific antigen-binding molecule according to claim 10, or the immune effector cell according to claim 12, or the nucleic acid fragment according to claim 13, or the vector according to claim 14, or the host cell according to claim 15, or an antibody or an antigen-binding fragment thereof or a multispecific antigen-binding molecule prepared by the method according to claim 16, or an immune effector cell prepared by the method according to claim 17, or the pharmaceutical composition according to claim 18 in preparing a medicament for preventing and/or treating a tumor, wherein the tumor is selected from a solid tumor, a hematologic tumor, or a B7H3-expressing infiltrative cancer;
preferably, the tumor is selected from melanoma, prostate cancer, renal cell carcinoma, lung cancer (e.g., non-small cell lung cancer (NSCLC)), and other solid tumors.

20. A method for preventing and/or treating a tumor, comprising administering to a patient in need thereof an effective amount of the antibody or the antigen-binding fragment thereof according to any one of claims 1-9, or the multispecific antigen-binding molecule according to claim 10, or the immune effector cell according to claim 12, or the nucleic acid fragment according to claim 13, or the vector according to claim 14, or the host cell according to claim 15, or an antibody or an antigen-binding fragment thereof or a multispecific antigen-binding molecule prepared by the method according to claim 16, or an immune effector cell prepared by the method according to claim 17, or the pharmaceutical composition according to claim 18, wherein the tumor is selected from a solid tumor, a hematologic tumor, or a B7H3-expressing infiltrative cancer;
preferably, the tumor is selected from melanoma, prostate cancer, renal cell carcinoma, lung cancer (e.g., non-small cell lung cancer (NSCLC)), and other solid tumors.

21. A kit, comprising the antibody or the antigen-binding fragment thereof according to any one of claims 1-9, or the multispecific antigen-binding molecule according to claim 10, or the immune effector cell according to claim 12, or the nucleic acid fragment according to claim 13, or the vector according to claim 14, or the host cell according to claim 15, or an antibody or an antigen-binding fragment thereof or a multispecific antigen-binding molecule prepared by the method according to claim 16, or an immune effector cell prepared by the method according to claim 17, or the pharmaceutical composition according to claim 18.

22. A method for detecting expression of B7H3 *in vitro,* comprising contacting a test sample with the antibody or the antigen-binding fragment thereof according to any one of claims 1-9 under conditions that allow the antibody or the antigen-binding fragment thereof according to any one of claims 1-9 and B7H3 to form a complex, wherein preferably, the method further comprises detecting formation of the complex and indicating the presence or expression level of DLL3 in the sample.

23. Use of the antibody or the antigen-binding fragment thereof according to any one of claims 1-9 in preparing a B7H3 detection reagent.
